(19) Europäisches Patentamt
European Patent Office
Office européen des brevets

(11) **EP 3 020 393 B1**

(12) **EUROPEAN PATENT SPECIFICATION**

(45) Date of publication and mention
of the grant of the patent:
**07.10.2020 Bulletin 2020/41**

(51) Int Cl.:
***A61K 9/14*** *(2006.01)*

(21) Application number: **15185026.0**

(22) Date of filing: **08.12.2010**

(54) **FORMULATIONS AND METHODS FOR CONTROLLING MDI PARTICLE SIZE DELIVERY**

FORMULIERUNGEN UND VERFAHREN ZUR STEUERUNG DER MDI-PARTIKELGRÖSSENFREISETZUNG

FORMULATIONS ET PROCÉDÉS DE COMMANDE DE DISTRIBUTION GRANULOMÉTRIQUE MDI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.12.2009 US 287072 P**

(43) Date of publication of application:
**18.05.2016 Bulletin 2016/20**

(62) Document number(s) of the earlier application(s) in
accordance with Art. 76 EPC:
**10790710.7 / 2 512 438**

(73) Proprietor: **3M Innovative Properties Company
St. Paul, MN 55133-3427 (US)**

(72) Inventor: **STEIN, Stephen W.
St. Paul, MN Minnesota 33427 (US)**

(74) Representative: **Vossius & Partner
Patentanwälte Rechtsanwälte mbB
P.O. Box 86 07 67
81634 München (DE)**

(56) References cited:
**WO-A1-03/086349     WO-A2-2009/095681**

**Description**

**Field**

The invention relates to metered dose inhalers and, in particular, to the medicinal aerosol formulations used in metered dose inhalers.

**Backround**

Metered Dose Inhalers (MDIs) are widely used for the treatment of respiratory diseases such as asthma, COPD, and allergic rhinitis. MDIs use high-pressure liquefied propellants to atomize the formulation into small droplets capable of delivering drug into the regions of the respiratory tract via oral or nasal inhalation. The drug(s) may be suspended in the formulation or may be dissolved to form a homogeneous solution.

For such aerosols, two key parameters that influence the deposition characteristics into the respiratory tract are the mass median aerodynamic diameter (MMAD) and the geometric standard deviation (GSD) of the delivered aerosol. The MMAD is a size measure of median diameter of the emitted particles, while the GSD is a measure of the span or size range distribution of the emitted particles. Many MDIs have particle sizes that do not get delivered to the desired location of the respiratory tract. It has not been uncommon, for example, to have 90% or more of the drug from an MDI delivered to the mouth and swallowed, instead of going into the lung. It is also important that drugs reach the correct location within the respiratory tract, such as the large and/or small airways of the lung, depending on the particular drug(s). The size distribution of the delivered drug particles in terms of MMAD and GSD is thus a key factor influencing the therapeutic effectiveness of MDI aerosols.

However, it is often difficult to control the MMAD and/or GSD for drugs in solution delivered from an MDI.

Moreover, some MDIs contain a combination of drugs where one drug is in solution and the other is in suspension. For example, an MDI for treatment of COPD formulated with an anticholinergic bronchidilator (ipratropium bromide) in solution and albuterol sulfate in suspension is described in WO99/65464. For such suspensionsolution combination products it can be even more challenging to achieve delivery of the drugs to a desired location in the respiratory tract.

**Summary**

It has now been found that the particle size distribution emitted from a solution or solution-suspension MDI formulation can be significantly modified to achieve desired airway deposition characteristics. The MMAD and/or GSD emitted from a solution-only MDI formulation (i.e., where all the drug is in dissolved form) can be modified by adding a non-drug particulate bulking agent (e.g., lactose) to the solution formulation. This can be used to modify the deposition characteristics of the emitted dose so to, e.g., allow the dissolved drug to be deposited more broadly throughout the large and small passages of the airways if desired.

Moreover, it has further been found that the size distribution and, importantly, the co-deposition characteristics of drugs emitted from a combination suspension-solution MDI (having dissolved drug and solid particulate drug in suspension) can be enhanced by reducing the mass median diameter (MMD) of the suspended particulate drug to less than 1.7 microns mass median diameter (MMD). Suspension-solution formulations normally use conventionally micronized drug of, e.g., 2 to 5 micron MMD, but using extra-fine suspended drug particles of less than 1.7 micron MMD results in enhanced co-deposition of the drugs to the same parts of the respiratory tract. This can be particularly important with respect to drugs that work together at the same tissue location for the intended therapeutic benefit. This co-deposition effect further increases with an MMD of the suspended drug at about 1.4 microns or less, and is preferably at about 1 micron MMD or less.

MDIs containing formulations of the invention may include other ingredients besides propellant, at least one dissolved drug, and bulking agent or suspended drug. For example a polar adjuvant such as ethanol may serve several purposes including valve lubrication, surfactant solubilization, and drug solubilization, if necessary. Surfactants and other excipients may be used for these and other reasons as well. A low-volatility excipient (e.g. glycerol) can also be used, for example, in order to increase the size of the emitted particle droplets. These and other ingredients, such as organic and inorganic acids, can also be used to enhance the physical and chemical stability of drugs, particularly drugs in solution. Disclosures of various formulation ingredients that can be used in conjunction with the present application can be found in, e.g., the following U.S. patent publications: 7018618, 7601336, 6716414, 6451285, 6315985, 6290930, 6045778, 6004537, 5776433, 5676930, US2003-0147814, and US2003-0152521.

It may be particularly useful to control the particle size distribution emitted from certain drug combinations in MDI products. For example, Johnson, M., (2004), "Inhaled corticosteroid -long-acting b2-agonist synergism: therapeutic implications in human lung disease", Proceedings of Respiratory Drug Delivery IX, 99-108, showed that it is desirable to deliver a corticosteroid and a long-acting beta agonist to the same tissues of the respiratory tract. 5-LO inhibitors are particularly useful for treatment of lung cancer when co-delivered with an immune response modifier drug such as imidazoquinoline amine (see, US2005/0267145). Pulmonary vaccine delivery may be enhanced by co-delivery and co-deposition of a vaccine antigen and a vaccine adjuvant (e.g. an immune response modifier). It may be desirable to deliver a therapeutic agent for systemic delivery in combination with a permeation enhancing agent. Delivery of an anti-inflammatory drug (such as a steroid) may be delivered with another drug that is efficacious when delivered to the lung, but that causes local irritation in the lung. This combination may maintain the efficacy of the drug, while minimizing lung irritation.

Many other combination therapies where co-deposition in the lung is desired are envisioned, including different drug class combinations such as beta agonists (especially a long-acting beta agonist) and steroid, beta agonist and anticholinergic, adenosine A2A receptor agonist and anticholinergic, 5-LO inhibitors and immune response modifier drug, vaccine antigen and vaccine adjuvant. More specific exemplary combinations include, e.g., fluticasone propionate and salmeterol (or salt form such as salmeterol xinafoate), ciclesonide and formoterol (or salt form such as formoterol fumarate or formoterol formamide), budesonide and formoterol (or salt form such as formoterol fumarate or formoterol formamide), ipratropium (or salt form such as iptratropium bromide) and albuterol (or salt form such as albuterol sulfate), ipratropium (or salt form such as iptratropium bromide) and fenoterol (or salt form such as fenoterol hydrobromide), a lipoxygenase inhibitor and imidazoquinoline amine, insulin and DPPC (dipalmitoylphosphatidylcholine), mometasone (or salt form such as mometasone furoate) and formoterol (or salt form such as formoterol fumarate or formoterol formamide), salmeterol (or salt form such as salmeterol xinafoate) and formoterol (or salt form such as formoterol fumarate or formoterol formamide), carmoterol (or salt form such as carmoterol hydrochloride) and budesonide, S-salmeterol (or salt form such as salmeterol xinafoate) and formoterol (or salt form such as formoterol fumarate or formoterol formamide), beclomethasone dipropionate and albuterol (or salt form such as albuterol sulfate), beclomethasone dipropionate and formoterol (or salt form such as formoterol fumarate or formoterol formamide), ipratropium (or salt form such as iptratropium bromide) and levalbuterol (or salt form such as levalbuterol tartrate), and tiotropium (or salt form such as tiotropium bromide) and formoterol (or salt form such as formoterol fumarate or formoterol formamide) . It is understood that different salt forms, esters, solvates, and enantiomers can also be used.

Any suitable non-drug bulking agent can be used to modify the emitted particle size distribution of a solution formulation. Preferred bulking agents include lactose, DLalanine, ascorbic acid, glucose, sucrose, trehalose as well as their various hydrates, anomers and/or enantiomers. Lactose including its various forms, such as a-lactose monohydrate and -lactose and alanine are more preferred. Lactose, in particular in its αlactose monohydrate form, is most preferred as a bulking agent due to e.g. processing considerations. Other suitable bulking agents include other saccharides e.g. D-galactose, maltose, D(+)raffinose pentahydrate, sodium saccharin, polysaccharides e.g. starches, modified celluloses, dextrins or dextrans, other amino acids e.g. glycine, salts e.g. sodium chloride, calcium carbonate, sodium tartrate, calcium lactate, or other organic compounds e.g. urea or propyliodone. Proteins, such as human serum albumin may also be used as non-drug bulking agents, as may be lipids such as phosphatidylcholine.

The concentration (w/w%) of drug dissolved in a formulation is generally greater than about 0.01%, often greater than about 0.04%, and often less than about 1%. The concentration (w/w%) of drug suspended in a formulation is generally greater than about 0.01% , often greater than about 0.04%, and often less than about 1%. It should be noted that the effects of suspended drug on the delivery and deposition characteristics of the dissolved drug depends in part on the number of suspended particles per volume, so that both the particle size and concentration of suspended drug can affect the results. To achieve enhanced codeposition of the dissolved and suspended drugs, it is generally preferred that the number of suspended drug particles is at least $3 \times 10^9$ suspended particles per milliliter, and preferably at least $1 \times 10^{10}$ suspended particles per milliliter.

Accordingly, the present invention includes, but is not limited to, at least the following embodiments:

1. A metered dose inhaler containing an aerosol formulation comprising:

> propellant;
> at least one drug dissolved in the formulation;
> at least one other drug in undissolved particulate form suspended in the formulation and having a mass median diameter of less than 1.7 microns.

2. A method of increasing the codeposition characteristics of two or more drugs delivered from a metered dose inhaler aerosol, comprising:
providing in a propellant formulation at least one drug dissolved in the formulation, and at least one drug as a particulate suspension in formulation having a MMD of less than 1.7 microns.

3. The metered dose inhaler of embodiment 1 or method of embodiment 2, wherein the at least one drug dissolved in the formulation is selected from the group consisting of beclomethasone dipropionate, ciclesonide, flunisolide, budesonide, fluticasone propionate, ipratropium, and tiotropium, mometasone, formoterol, including any physiologically acceptable salt, solvate, or enantiomer of any of the foregoing.

4. The metered dose inhaler or method of any preceding embodiment, where the drug completely dissolved in the formulation is present at a concentration of at least 0.01% by weight of the formulation.

5. The metered dose inhaler or method of any preceding embodiment, wherein the propellant includes a compound selected from the group consisting of 1,1,1,2-tetrafluoroethane (HFC-134a), 1,1,1,2,3,3,3-heptafluoropropane (HFC-227), and mixtures thereof.

6. The metered dose inhaler or method of any preceding embodiment, wherein the at least one other drug in undissolved particulate form suspended in the formulation is selected from the group consisting of albuterol, formoterol, and salmeterol, including any physiologically acceptable salt, solvate, or enantiomer of any of the foregoing.

7. The metered dose inhaler or method of any preceding embodiment, wherein the at least one other drug in undissolved particulate form suspended in the formulation has a mass median diameter of no greater than 1.4 microns.

8. The metered dose inhaler or method of embodiment 6, wherein the mass median diameter is no greater than 1 micron.

9. The metered dose inhaler or method of any preceding embodiment, further comprising ethanol.

10. The metered dose inhaler or method of any preceding embodiment, further comprising a dispersing aid.

11. The metered dose inhaler or method of any preceding embodiment, wherein the number of suspended drug particles is at least $3 \times 10^9$ suspended particles per milliliter of formulation.

12. The metered dose inhaler or method of any preceding embodiment, wherein the number of suspended drug particles is at least $1 \times 10^{10}$ suspended particles per milliliter of formulation.

13. The metered dose inhaler or method of any preceding embodiment, further comprising a particulate bulking agent.

14. A metered dose inhaler containing an aerosol formulation comprising:

propellant;
one or more drugs all of which is in dissolved form;
at least one non-drug bulking agent in undissolved particulate form suspended in the formulation.

15. The metered dose inhaler of embodiment 14, wherein the drug is selected from the group consisting of beclomethasone dipropionate, fluticasone propionate, budesonide, mometasone, ciclesonide, flunisolide, ipratropium, and tiotropium, including any physiologically acceptable salt, solvate, or enantiomer of any of the foregoing.

16. The metered dose inhaler of embodiments 14 or 15, where the drug completely dissolved in the formulation is present at a concentration of at least 0.01% by weight of the formulation.

17. The metered dose inhaler of any of embodiments 14-16, wherein the propellant includes a compound selected from the group consisting of 1,1,1,2-tetrafluoroethane (HFC-134a), 1, 1, 1,2,3,3,3-heptafluoropropane (HFC-227), and mixtures thereof.

18. The metered dose inhaler of any of embodiments 14-17, wherein the bulking agent is selected from the group consisting of lactose, DL-alanine, ascorbic acid, glucose, sucrose, D(+)trehalose.

19. The metered dose inhaler of any of embodiments 14-18, further comprising ethanol.

20. The metered dose inhaler of any of embodiments 14-19, further comprising a dispersing aid.

21. The metered dose inhaler of any of embodiments 14-20, wherein the non-drug bulking agent has an MMD of at least 2 microns.

22. The metered dose inhaler or method of any preceding embodiment, further comprising a low-volatility excipient.

It should be noted to avoid confusion that as used herein reference to "particles" as emitted from an MDI (and, e.g., delivered to the lungs or nasal passages, or collected on plates of an Anderson Cascade Impactor) can include both solid particles and droplets of formulation containing still dissolved drug. However, reference to "particulate" and "particles" suspended in an MDI formulation refers to insoluble solid particles suspended in a formulation in an MDI container prior to delivery. This is a common usage of the terms in the MDI field.

All percentages are, unless otherwise indicated, given by weight relative to the weight of the formulation.

Also, mass median diameter (which is equivalent to volume median diameter) of the suspended particulate can be determined using any conventional particle size measurement method known to those skilled in the art. Suitable methods include for example laser diffraction, photon correlation spectroscopy (e.g. using a spectrometer available under the trade designation Brookhaven PCS from Brookhaven Inc.), spinning disc centrifuging (using an instrument available under the trade designation CPS Disc Centrifuge from Chemical Process Specialists Inc.) and scanning electron microscopy (SEM). Mass median diameter is preferably determined by laser diffraction, photon correlation spectroscopy or spinning disc centrifuging, more preferably by laser diffraction, more particularly laser diffraction using an analyser available under the trade designation Malvern Mastersizer 2000 laser light diffraction particle size analyzer from Malvern Instruments Ltd.

Various other features and advantages of the present invention should become readily apparent with reference to the following detailed description, examples, claims and appended drawings. In several places throughout the specification, guidance is provided through lists of examples. In each instance, the recited list serves only as a representative group and should not be interpreted as an exclusive list.

**Brief Description of the Drawings**

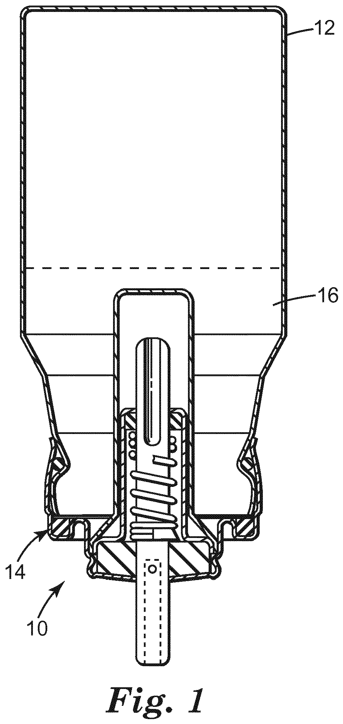

Fig. 1 is a cross-sectional diagram of a metered dose inhaler of a kind that may be used with the present invention.

**Detailed Description**

Fig. 1 shows a typical metered dose inhaler 10 having a canister 12 equipped with a metering valve 14 and containing a medicinal aerosol formulation 16 that can be used according to the present invention. The metered dose inhaler also normally includes an actuator or adaptor (not shown) that is used to deliver the aerosol formulation to the lungs via oral inhalation or nasal passages via nasal inhalation.

Formulations of the invention contain a propellant (preferably HFA-134a or HFA227), a drug dissolved in the formulation, solid particles (of drug or bulking agent) suspended in the formulation, and optionally a cosolvent (e.g. ethanol), optionally a surfactant, and optionally other excipients. The size distribution and concentration of the suspended drug or bulking agent particles can be varied depending on the emitted particle size distribution desired for the dissolved drug.

Surprisingly, smaller suspended particles are often more effective at increasing the MMAD of the emitted dissolved drug than are larger suspended particles. When suspended particles are another drug, smaller size can provide enhanced co-deposition. Suspended particles with an MMD less than 1.7 microns are good at increasing the MMAD and increasing co-deposition, with particles having an MMD 1.4 microns or less being better, and particles with an MMD 1 micron or less being particularly preferred. Suspended particles with an MMD 0.5 microns and less are also quite effective at doing this.

Larger suspended particles (e.g., conventionally micronized to 2-5 microns MMD) of non-drug bulking agent result in less increase in the MMAD of dissolved drug emitted from the MDI, but can significantly increase the spread of the distribution as represented by an increase in the GSD. A larger GSD may be desirable to provide broader deposition of a solution-only formulation throughout the lungs, and a bulking agent having particles of 2 to 5 microns may be particularly useful for this purpose.

When a non-drug bulking agent is used with drug in solution, it may be any physiologically acceptable insoluble particulate that is sufficiently stable in the formulation at the desired size range. Sugars such as lactose, sucrose, or trehalose are examples. Preferred bulking agents include lactose, DL-alanine, ascorbic acid, glucose, sucrose, trehalose as well as their various hydrates, anomers and/or enantiomers. Lactose including its various forms, such as a-lactose monohydrate and -lactose and alanine are more preferred. Lactose, in particular in its a-lactose monohydrate form, is most preferred as a bulking agent due to e.g. processing considerations. Other suitable bulking agents include other saccharides e.g. D-galactose, maltose, D(+)raffinose pentahydrate, sodium saccharin, polysaccharides e.g. starches, modified celluloses, dextrins or dextrans, other amino acids e.g. glycine, salts e.g. sodium chloride, calcium carbonate, sodium tartrate, calcium lactate, or other organic compounds e.g. urea or propyliodone. Proteins, such as human serum albumin may also be used as non-drug bulking agents, as may be lipids such as phosphatidylcholine or DPPC (which can also be used therapeutically in higher concentrations as a lung surfactant).

The solid particles of non-drug bulking agent should be suspended in a concentration and with a size distribution sufficient to substantially alter the size distribution or deposition profile of the solubilized drug. In-vitro assessments may be used to determine if the size distribution has been substantially altered. This is often done using aerodynamic size distribution tests such as cascade impactor (CI) testing. A 'substantial difference' in the size distribution may be, for example, at least a 10% increase in the MMAD or change in the GSD of at least 0.1. In many cases, the change in MMAD when a solid particle bulking agent is used may be, e.g. 20 or 30%. In many cases, a change in GSD when the solid particle bulking agent is used may be 0.2 or more.

When the formulation uses one drug in suspension and one in solution, similar particle size distributions and thus co-deposition for the two drugs can be achieved when particles of the suspended drug have an MMD of less than about 1.7 microns, preferably about 1.4 microns or less, and most preferably 1.0 microns or less. The formulations may contain a cosolvent, surfactant and other excipients. These formulations are contained in an MDI canister with a metering valve crimped on to it, and are delivered using an MDI adaptor or actuator for oral or nasal inhalation.

The pharmaceutical formulations according to the invention may contain one or more drugs (therapeutically or prophylactically active compounds), for example selected from anti-inflammatory agents, anticholinergic agents (particularly an $M_1$, $M_2$, $M_1/M_2$ or $M_3$ receptor antagonist), $\beta_2$-adrenoreceptor agonists, antiinfective agents (e.g. antibiotics, antivirals), antihistamines, as well as vaccines and vaccine adjuvants.

The invention thus provides, in a further aspect, a pharmaceutical aerosol formulation as described herein with one or more therapeutically active agents, for example, selected from an anti-inflammatory agent (for example a corticosteroid or an NSAID), an anticholinergic agent, a $\beta_2$-adrenoreceptor agonist, an antiinfective agent (e.g. an antibiotic or an antiviral), an antihistamine, or a vaccine and vaccine adjuvant.

Preferred combinations include a corticosteroid with a long-acting beta agonist or anticholinergic, a vaccine antigen with a vaccine adjuvant, a beta agonist with an anticholinergic, and an adenosine A2A receptor agonist with an anticholinergic.

Examples of $\beta_2$-adrenoreceptor agonists include salmeterol (e.g. as racemate or a single enantiomer such as the R-enantiomer or the S-enantiomer), salbutamol (e.g. as racemate or a single enantiomer such as the R-enantiomer), formoterol (e.g. as racemate or a single enantiomer such as the R,R-enantiomer), fenoterol, carmoterol, etanterol, naminterol, clenbuterol, pirbuterol, flerbuterol, reproterol, bambuterol, terbutaline salmefamol, indacaterol and salts thereof, for example the xinafoate (1-hydroxy-2naphthalenecarboxylate) salt of salmeterol, the sulphate salt of salbutamol or the fumarate salt of formoterol. Long-acting $\beta_2$-adrenoreceptor agonists, for example, compounds which provide effective bronchodilation for about 12 hours or longer, are preferred. Other $\beta_2$-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WOO1142193 and WO03/042160.

Particular $\beta_2$-adrenoreceptor agonists include:

3-(4-{[6-({(2R)-2-hydroxy-2-[4-hydroxy-3-(hydroxymethyl)phenyl]ethyl}amino)hexyl]oxy}butyl)benzenesulfonamide;

3-(3-{[7-({(2R)-2-hydroxy-2-[4-hydroxy-3-hydroxymethyl)phenyl]ethyl}-amino)heptyl]oxy}propyl) benzenesulfonamide;

4-{(IR)-2-[(6-{2-[(2,6-dichlorobenzyl)oxy]ethoxy}hexyl)amino]-I-hydroxyethyl}-2-(hydroxymethyl)phenol;

4-{(IR)-2-[(6-{4-[3-(cyclopentylsulfonyl)phenyl]butoxy} hexyl)amino]-I-hydroxyethyl}2-(hydroxymethyl)phenol;

N-[2-hydroxyl-5-[(IR)-I-hydroxy-2-[[2-4-[[(2R)-2-hydroxy-2phenylethyl] amino]phenyl]ethyl]amino]ethyl]phenyl]formamide;

N-{2-[4-(3-phenyl-4-methoxyphenyl)aminophenyl]ethyl}-2-hydroxy-2-(8-hydroxy-2(1H)-quinolinon-5-yl)ethylamine; and

5-[(R)-2-(2-{4-[4-(2-amino-2-methyl-propoxy)-phenylamino]-phenyl}-ethylamino)-1-hydroxy-ethyl]-8-hydroxy-1H-quinolin-2-one;

and pharmaceutically acceptable salts thereof.

The $\beta_2$-adrenoreceptor agonist may be in the form of a salt formed with a pharmaceutically acceptable acid selected from sulphuric, hydrochloric, fumaric, hydroxynaphthoic (for example 1-or 3-hydroxy-2-naphthoic), cinnamic, substituted cinnamic, triphenylacetic, sulphamic, sulphanilic, naphthaleneacrylic, benzoic, 4-methoxybenzoic, 2-or 4-hydroxybenzoic, 4-chlorobenzoic and 4-phenylbenzoic acid.

Suitable anti-inflammatory agents include corticosteroids. Suitable corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, $6\alpha,9\alpha$-difluoro-11$\beta$-hydroxy-16$\alpha$-methyl-17$\alpha$-[(4-methyl-1,3-thiazole-5carbonyl)oxy]-3-oxo-androsta-1,4-diene-17$\beta$-carbothioic acid S-fluoromethyl ester, $6\alpha,9\alpha$-difluoro-11$\beta$-hydroxy-16$\alpha$-methyl-3-oxo-17$\alpha$-propionyloxy-androsta-1,4-diene17$\beta$-carbothioic acid S-(2-oxo-tetrahydro-furan-3S-yl)ester, $6\alpha,9\alpha$-difluoro-11$\beta$-hydroxy16$\alpha$-methyl-3-oxo-17$\alpha$-(2,2,3,3-tetramethycyclopropylcarbonyl)oxy-androsta-1,4-diene17$\beta$-carbothioic acid Scyanomethyl ester, $6\alpha,9\alpha$-difluoro-11$\beta$-hydroxy-16$\alpha$-methyl-17$\alpha$-(I-methycyclopropylcarbonyl)oxy-3-oxo-androsta-1,4-diene-17$\beta$-carbothioic acid Sfluoromethyl ester, beclomethasone esters (eg. the 17-propionate ester or the 17,21dipropionate ester), budesonide, flunisolide, mometasone esters (eg. the furoate ester), triamcinolone acetonide, rofleponide, ciclesonide (16$\alpha$, 17-[[(R)cyclohexylmethylene ]bis(oxy)]-11$\beta$,21-dihydroxy-pregna-1,4-diene-3,20-dione), butixocort propionate, RPR-I06541, and ST-126. Preferred corticosteroids include fluticasone propionate, $6\alpha,9\alpha$-difluoro-11$\beta$-hydroxy-16$\alpha$-methyl-17$\alpha$-[(4-methyl-1,3thiazole-5-carbonyl)oxy]-3-oxo-androsta-1,4-diene-17$\beta$-carbothioic acid S-fluoromethyl ester, $6\alpha,9\alpha$-difluoro-11$\beta$-hydroxy-16$\alpha$-methyl-3-oxo-17$\alpha$-(2,2,3,3-tetramethylcyclopropylcarbonyl)oxy-androsta-1,4-diene-17$\beta$-carbothioic acid Scyanomethyl ester and $6\alpha,9\alpha$-difluoro-11$\beta$-hydroxy-16$\alpha\beta$-methyl-17$\alpha$,-(1methycyclopropylcarbonyl)oxy-

3-oxo-androsta-1,4-diene-17β-carbothioic acid Sfluoromethyl ester.

Non-steroidal compounds having glucocorticoid agonism that may possess selectivity for transrepression over transactivation and that may be useful in combination therapy include those covered in the following patents: WO03/082827, WOO1110143, WO98/54159, WO04/005229, WO04/009016, WO04/009017, WO04/018429, WO031104195, WO03/082787, WO03/082280, WO03/059899, WO031101932, WO02/02565, WO01116128, WO00/66590, WO03/086294, WO04/026248, WO03/06 1651 and WO03/08277.

Suitable anti-inflammatory agents include non-steroidal anti-inflammatory drugs (NSAID' s). Suitable NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS inhibitors, tryptase and elastase inhibitors, beta-2 integrin antagonists and adenosine receptor agonists or antagonists (e.g. adenosine 2a agonists), cytokine antagonists (e.g. chemokine antagonists, such as a CCR3 antagonist) or inhibitors of cytokine synthesis, or 5-lipoxygenase inhibitors. An iNOS (inducible nitric oxide synthase inhibitor) is preferably for oral administration. Suitable iNOS inhibitors include those disclosed in WO93/13055, WO98/30537, WO02/50021, WO95/34534 and WO99/62875. Suitable CCR3 inhibitors include those disclosed in WO02/26722.

Combinations including a phosphodiesterase 4 (PDE4) inhibitor may be used. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds of interest include *cis*-4-cyano-4-(3-cyclopentyloxy-4-methoxyphenyl)cyclohexan-1-carboxylic acid, 2-carbomethoxy-4-cyano-4-(3 cyclopropylmethoxy-4-difluoromethoxyphenyl)cyclohexan-1-one and *cis*-[4-cyano-4-(3cyclopropylmethoxy-4-difluoromethoxyphenyl)cyclohexan-1-ol]. Also, *cis*-4-cyano-4-[3(cyclopentyloxy)-4-methoxyphenyl ]cyclohexane-1-carboxylic acid (also known as cilomilast) and its salts, esters, pro-drugs or physical forms, which is described in U.S. patent 5,552,438.

Other compounds of interest include A WD-12-281 from Elbion (Hofgen, N. et al. 15th EFMC lnt Symp Med Chern (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as Cl-I018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p-[(4aR *,1 ObS *)-9ethoxy-1,2,3,4,4a, 1 Ob-hexahydro-8-methoxy-2-methylbenzo[c] [1 ,6]naphthyridin-6-yl]N,N-diisopropylbenzamide which is a mixed PDE3/PDE4 inhibitor which has been prepared and published on by Byk-Gulden, now Nycomed; arofylline under development by Almirall-Prodesfarma; VM554IUM565 from Vernalis; or T-440 (Tanabe Seiyaku; Fuji, K. et al. J Pharmacol Exp Ther,1998, 284(1): 162), and T2585. Further compounds of interest are disclosed in the published international patent application WO04/024728 (Glaxo Group Ltd), PCT/EP2003/014867 (Glaxo Group Ltd) and PCT/EP2004/005494 (Glaxo Group Ltd).

Suitable anticholinergic agents are those compounds that act as antagonists at the muscarinic receptors, in particular those compounds which are antagonists of the M1 or M3 receptors, dual antagonists of the MdM3 or M2/M3, receptors or pan-antagonists of the M1/M2/M3 receptors. Exemplary compounds for administration via inhalation include ipratropium (e.g. as the bromide, CAS 22254-24-6, sold under the name Atrovent), oxitropium (e.g. as the bromide, CAS 30286-75-0) and tiotropium (e.g. as the bromide, CAS 136310-93-5, sold under the name Spiriva). Also of interest are revatropate (e.g. as the hydrobromide, CAS 262586-79-8) and LAS-34273 which is disclosed in WOO1/04118. Exemplary compounds for oral administration include pirenzepine (CAS 28797-61-7), darifenacin (CAS 133099-04-4, or CAS 133099-07-7 for the hydrobromide sold under the name Enablex), oxybutynin (CAS 5633-20-5, sold under the name Ditropan), terodiline (CAS 15793-40-5), tolterodine (CAS 124937-51-5, or CAS 12493752-6 for the tartrate, sold under the name Detrol), otilonium (e.g. as the bromide, CAS 26095-59-0, sold under the name Spasmomen), trospium chloride (CAS 10405-02-4) and solifenacin (CAS 242478-37-1, or CAS 242478-38-2 for the succinate also known as YM905 and sold under the name Vesicare).

Suitable antihistamines (also referred to as HI-receptor antagonists) include any one or more of the numerous antagonists known which inhibit HI-receptors, and are safe for human use. First generation antagonists, include derivatives of ethanolamines, ethylenediamines, and alkylamines, e.g diphenylhydramine, pyrilamine, clemastine, chloropheniramine. Second generation antagonists, which are non-sedating, include loratidine, desloratidine,terfenadine,astemizole,acrivastine, azelastine, levocetirizine fexofenadine and cetirizine. Examples of preferred anti-histamines include loratidine, desloratidine, fexofenadine and cetirizine.

Particular combinations include, for example, dissolved ipratropium bromide with suspended albuterol sulfate, dissolved ciclesonide with suspended formoterol fumarate, dissolved budesonide with suspended formoterol fumarate, dissolved ipratropium bromide with suspended formoterol fumarate, dissolved fluticasone propionate with suspended salmeterol xinafoate, and dissolved ipratropium bromide with suspended salmeterol xinafoate.

**Experimental Examples**

The following examples have been selected merely to further illustrate features, advantages, and other details of the invention. It is to be expressly understood, however, that while the examples serve this purpose, the particular materials and amounts used as well as other conditions and details are not to be construed in a matter that would unduly limit the scope of this invention.

Example 1

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and an actuator as used on the commercially available MDI product QVAR™ (commercially available from Teva Specialty Pharmaceuticals LLC) was filled with a formulation containing HFA-134a (91.5% by weight), oleic aid (0.018% by weight), ethanol (8.0% by weight), beclomethasone dipropionate (0.084% by weight) dissolved in the formulation, and size reduced albuterol sulfate (0.402% by weight) suspended in the formulation. The size reduced albuterol sulfate was prepared by processing 12.0573 grams of micronized albuterol sulfate in 150 ml of 200 proof ethanol in an Avestin EmulsiFlex-C50 high shear homogenizer (Avestin Europe GmbH, Mannheim, Germany) and processing at 10 kpsi for 4 minutes and then processing at 15 kpsi for 5 minutes and then processing at 18 kpsi for 10 minutes to provide a mass median particle diameter (MMD) of 1.06 microns. The MMD was measured using a Malvern Mastersizer 2000.

Example 2

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVAR™ actuator (commercially available from Teva Specialty Pharmaceuticals LLC) was filled with a formulation containing HFA-134a (91.6% by weight), oleic aid (0.017% by weight), ethanol (8.1% by weight), flunisolide hemihydrate (0. 171% by weight) dissolved in the formulation, and size reduced albuterol sulfate (0.126% by weight) suspended in the formulation. The size reduced albuterol sulfate was prepared by processing 12.0573 grams of micronized albuterol sulfate in 150 ml of 200 proof ethanol in an Avestin EmulsiFlex-C50 high shear homogenizer (Avestin Europe GmbH, Mannheim, Germany) and processing at 10 kpsi for 4 minutes and then processing at 15 kpsi for 5 minutes and then processing at 18 kpsi for 10 minutes to provide a mass median particle diameter (MMD) of 1.06 microns. The MMD was measured using a Malvern Mastersizer 2000.

Comparative Example 1

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVAR™ actuator (commercially available from Teva Specialty Pharmaceuticals LLC) was filled with a formulation containing HF A -134a (91.6% by weight), oleic aid (0.030% by weight), ethanol (7.9% by weight), beclomethasone dipropionate (0.083% by weight of dissolved material), and micronized albuterol sulfate (0.397% of suspended material). The micronized albuterol sulfate was micronized using a conventional air jet mill to provide a mass median particle diameter (MMD) of 1.70 microns. The MMD was measured using a Malvern Mastersizer 2000.

Comparative Example 2

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVARTM actuator (commercially available from Teva Specialty Pharmaceuticals LLC) was filled with a formulation containing HFA-134a (91.6% by weight), oleic aid (0.020% by weight), ethanol (8.0% by weight), flunisolide hemihydrate (0.169% by weight of dissolved material), and micronized albuterol sulfate (0.121% of suspended material). The micronized albuterol sulfate was micronized using a conventional air jet mill to provide a mass median particle diameter (MMD) of 1.70 microns. The MMD was measured using a Malvern Mastersizer 2000.

Example 3

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVARTM actuator (commercially available from Teva Specialty Pharmaceuticals LLC) was filled with a formulation containing HFA-134a (89.1% by weight), ethanol (9.9% by weight), beclomethasone dipropionate (0.080% by weight) dissolved in the formulation, and size reduced lactose monohydrate (0.973% by weight) suspended in the formulation. The size reduced lactose monohydrate was prepared by processing 11.9948 grams of micronized lactose monohydrate in 121.9097 grams of 200 proof ethanol in an Avestin EmulsiFlex-C50 high shear homogenizer (Avestin Europe GmbH, Mannheim, Germany) and processing 15 minutes to provide a mass median particle diameter (MMD) of 1.06 microns. Details on the procedure for reducing the size of lactose monohydrate using the EmulsiFlex-C50 high shear homogenizer can be found in (US200408 1627). The MMD was measured using a Malvern Mastersizer 2000.

Example 4

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVAR™ actuator (commercially available from Teva Specialty Pharmaceuticals LLC) was filled with a formulation containing HF A-134a (90.9% by weight), ethanol (8.0% by weight), beclomethasone dipropionate (0.085% by weight) dissolved in the formulation, and micronized lactose monohydrate (1.003% by weight) suspended in the formulation. The micronized lactose monohydrate had a mass median particle diameter (MMD) of 2.12 microns. The MMD was measured using a Malvern Mastersizer 2000.

Example 5

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVAR™ actuator (commercially available from Teva Specialty Pharmaceuticals LLC) was filled with a formulation containing HFA-134a (91.7% by weight), ethanol (8.0% by weight), oleic acid (0.021 % by weight), flunisolide hemihydrate (0.169% by weight) dissolved in the formulation, and size reduced lactose monohydrate (0.101 % by weight) suspended in the formulation. The size reduced lactose monohydrate was prepared by processing 11.9948 grams of micronized lactose monohydrate in 121.9097 grams of 200 proof ethanol in an A vestin EmulsiFlex-C50 high shear homogenizer (Avestin Europe GmbH, Mannheim, Germany) and processing 15 minutes to provide a mass median particle diameter (MMD) of 1.06 microns. Details on the procedure for reducing the size of lactose monohydrate using the EmulsiFlex-C50 high shear homogenizer can be found in (US2004081627). The MMD was measured using a Malvern Mastersizer 2000.

Example 6

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVARTM actuator (commercially available from Teva Specialty Pharmaceuticals LLC) was filled with a formulation containing HFA-134a (91.0% by weight), ethanol (7.9% by weight), oleic acid (0.019% by weight), flunisolide hemihydrate (0.164% by weight) dissolved in the formulation, and micronized lactose monohydrate (0.987% by weight) suspended in the formulation. The micronized lactose monohydrate had a mass median particle diameter (MMD) of 2.12 microns. The MMD was measured using a Malvern Mastersizer 2000.

Comparative Example 3

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVARTM actuator (commercially available from Teva Specialty Pharmaceuticals LLC) was filled with a formulation containing HFA-134a (91.9% by weight), ethanol (8.0% by weight), and beclomethasone dipropionate (0.083% by weight of dissolved material).

Comparative Example 4

An experimental metered dose inhaler (MDI) composed of a 14 ml deep drawn aluminum canister (3M-Neotechnic Ltd., Clitheroe, UK); a 50 microliter SPRAYMISER valve (3M-Neotechnic Ltd., Clitheroe, UK) and a QVARTM actuator (commercially available from Teva Specialty Pharmaceuticals LLC) was filled with a formulation containing HFA-134a (91.8% by weight), oleic aid (0.019% by weight), ethanol (8.0% by weight), and flunisolide hemihydrate (0.166% by weight of dissolved material).

Anderson Cascade Impactor (ACI) Studies for Examples 1 and 2 and Comparative Examples 1 and 2

The aerodynamic particle size distribution emitted from each MDI was evaluated using the Andersen Mark II Cascade Impactor (ACI) (Thermo Fisher Scientific, Waltham, Massachusetts). Three Andersen cascade impactor (ACI) tests were conducted on each of the formulations by coupling the MDI to a USP inlet ('Throat') and actuating five times into the ACI setup. The flow rate during testing was 28.3 liters per minute (lpm). The drug collected on the valve stem, actuator, Throat, jet for Stage 0 of the ACI, all of the ACI impaction plates (plates 0-7), and the filter was determined by rinsing each individual section with a known volume of solvent (3:1 ratio of methanol: 0.1% phosphoric acid in water) and then analyzing the recovered samples using an HPLC assay with a reference standard curve. The impaction plates of the ACI were not coated for any of the tests.

Tables 1-4 list the amount of sample (mcg/dose) recovered at each stage of the ACI for Examples 1-2 and Comparative Examples 1-2. The results are averages of three separate impactor tests. The degree of co-deposition of the suspended and dissolved drugs in each example was determined by plotting the amount of dissolved drug deposited on each stage of the ACI to the amount of suspended drug deposited on the same stage of the ACI and determining the R-squared value of the plots. R-Squared, or determination coefficient, is a statistical term indicating the degree to which two values correlate. Only the impactor stages (Jet Stage 0 thru Filter) were used to determine the R-squared calculation (deposition data for the valve stem, actuator, and USP inlet was not included in the calculation). Table 5 lists for each Example and Comparative Example the corresponding mass median aerodynamic diameter (MMAD) and geometric standard deviation (GSD) of the delivered aerosol measured by the ACI for both the drug that was suspended in the formulation and the drug that was dissolved in the formulation. The MMAD and GSD values were calculated from the ACI measurements for drug entering into the impactor using the DISTFIT VERSION 2 software program (Chimera Technologies, Forest Lake, MN). The size distributions were fit using a mono-modal distribution assumption. Other approaches and software could be used to estimate the central tendency and spread of the delivered size distribution. The R-squared values from Tables 1-4 are included again in Table 5 for clarity purposes. The concentration of suspended drug particles in the formulation was calculated using equation 1 (adapted from Stein, S.W. AAPSPharmSciTech (2008),19,112-115). MMD is the mass median diameter of the micronized drug to be suspended in the formulation (in centimeters). GSD is the geometric standard deviation of the micronized drug to be suspended in the formulation (dimensionless). $C_D$ is the mass concentration of drug in the formulation (in grams per milliliter). $C_p$ is the concentration of suspended drug particles in the formulation (in particles per milliliter), the particle density is represented as $\rho$ (in grams per milliliter). A MALVERN MASTERSIZER 2000 (Malvern Instruments, Westborough, MA) was used to determine the MMD and GSD values of the micronized drug suspended in the formulation. In this method the instrument measured the input drug size from a slurry of the drug dispersed in a solution of 0.7% Span 85 in heptane.

$$C_P = \frac{6C_D \exp(4.5\ln^2 GSD)}{\rho\pi(MMD)^3}$$

Equation 1

Anderson Cascade Impactor (ACI) Studies for Examples 3 through 6 and Comparative Examples 3 and 4

The aerodynamic particle size distribution from each MDI was evaluated using the Andersen Mark II Cascade Impactor (ACI) (Thermo Fisher Scientific, Waltham, Massachusetts). At least three Andersen cascade impactor (ACI) tests were conducted on each of the formulations by coupling the MDI to a USP inlet ('Throat') and actuating five times into the ACI setup. The flowrate during testing was 28.3 liters per minute (lpm). The drug collected on the valve stem, actuator, Throat, jet for Stage 0 of the ACI, and all of the ACI impaction plates (plates 0-7), and the filter was determined by rinsing each individual section with a known volume of solvent (methanol) to dissolve the drug. The samples were filtered using Acrodisc® 45 micron PTFE membrane filter (PALL Life Sciences, Port Washington, NY) to remove any undissolved lactose particles. The filtrate for each sample was then analyzed using an HPLC assay with a reference standard curve to determine the amount of drug deposited on each component of the test setup. The impaction plates of the ACI were not coated for any of the tests.

Tables 6-8 list the amount of sample (mcg/dose) recovered at each stage of the ACI for Examples 3-6 and Comparative Examples 3-4. The results are averages of three separate impactor tests. Table 9 lists for Examples 3-6 and Comparative Examples 3-4 the corresponding mass median aerodynamic diameter (MMAD) and geometric standard deviation (GSD) of the dissolved drug from the delivered aerosol measured by the ACI. The MMAD and GSD values were calculated from the ACI measurements for drug entering into the impactor using the DISTFIT VERSION 2 software program (Chimera Technologies, Forest Lake, MN). The size distributions were fit using a mono-modal distribution assumption. Other approaches and software could be used to estimate the central tendency and spread of the delivered size distribution. A MALVERN MASTERSIZER 2000 (Malvern Instruments, Westborough, MA)) was used to determine the MMD and GSD values of the lactose excipient suspended in the formulation. In this method the instrument measured the input excipient size from a slurry of the lactose dispersed in a solution of 0.7% Span 85 in heptane.

Table 1: ACI Results for Example 1

| Test Component | Albuterol Sulfate (mcg/dose) | *Beclomethasone Dipropionate* |
|---|---|---|
| Valve Stem | 17.99 | *3.65* |
| Actuator | 57.15 | *11.78* |
| Throat (USP Inlet) | 54.32 | *11.08* |
| Jet for Stage 0 | 0.39 | *0.66* |
| Plate 0 | 1.37 | *0.12* |
| Plate 1 | 0.85 | *0.05* |
| Plate 2 | 1.93 | *0.25* |
| Plate 3 | 8.82 | *1.67* |
| Plate 4 | 27.06 | *4.85* |
| Plate 5 | 47.65 | *7.96* |
| Plate 6 | 18.24 | *3.30* |
| Plate 7 | 3.13 | *1.72* |
| *Filter* | *4.24* | *2.16* |
| R-squared value for drug entering impactor (Jet Stage thru Filter): 0.955 | | |

Table 2: ACI results for Example 2

| Test Component | Albuterol Sulfate (mcg/dose) | *Flunisolide Hemihydrate* |
|---|---|---|
| Valve Stem | 4.85 | 5.26 |
| Actuator | 16.03 | *21.93* |
| Throat (USP Inlet) | 15.84 | *23.75* |
| Jet for Stage 0 | 0.12 | *0.18* |
| Plate 0 | 0.57 | *0.34* |
| Plate 1 | 0.09 | *0.26* |
| Plate 2 | 0.14 | *0.29* |
| Plate 3 | 1.88 | *2.47* |
| Plate 4 | 6.97 | *7.43* |
| Plate 5 | 15.63 | *18.37* |
| Plate 6 | 5.05 | *9.28* |
| Plate 7 | 0.11 | *4.53* |
| *Filter* | *0.11* | *6.30* |
| R-squared value for drug entering impactor (Jet Stage thru Filter): 0.841 | | |

Table 3: ACI Results for Comparative Example 1

| Test Component | Albuterol Sulfate (mcg/dose) | Beclomethasone Dipropionate |
|---|---|---|
| Valve Stem | 13 .79 | *2.31* |
| Actuator | 72. 17 | *11.53* |

(continued)

| Test Component | Albuterol Sulfate (mcg/dose) | Beclomethasone Dipropionate |
|---|---|---|
| Throat (USP Inlet) | 60.33 | *9.54* |
| Jet for Stage 0 | 1.26 | *0.14* |
| Plate 0 | 3.22 | *0.31* |
| Plate 1 | 3.21 | *0.21* |
| Plate 2 | 5.87 | *0.41* |
| Plate 3 | 21.35 | *1.97* |
| Plate 4 | 42.23 | *4.45* |
| Plate 5 | 29.33 | *6.81* |
| Plate 6 | 4.43 | *4.34* 15 |
| Plate 7 | 1.23 | *3.24* |
| Filter | 2.26 | *2.58* |
| R-squared value for drug entering impactor (Jet Stage thru Filter): 0.385 | | |

Table 4: ACI Results for Comparative Example 2

| Test Component | Albuterol Sulfate (mcg/dose) | Flunisolide *Hemihydrate* |
|---|---|---|
| Valve Stem | 3.54 | *4.16* |
| Actuator | 20.11 | *23.30* |
| Throat (USP Inlet) | 18.84 | *20.45* |
| Jet for Stage 0 | 0.55 | *0.34* |
| Plate 0 | 1.21 | *0.76* |
| Plate 1 | 1.1 0 | *0.40* |
| Plate 2 | 1.72 | *0.69* |
| Plate 3 | 5.24 | *3.21* |
| Plate 4 | 11.43 | *8.68* |
| Plate 5 | 10.48 | *17.69* |
| Plate 6 | 2.09 | *9.94* |
| Plate 7 | 0.00 | *5.36* |
| Filter | *0.00* | *4.69* |
| R-squared value for drug entering impactor (Jet Stage thru Filter): 0.487 | | |

Table 5

| | Dissolved Drug MMAD (microns) | Dissolved Drug GSD | Suspended DrugMMAD (microns) | Suspended Drug GSD | Concentration of Suspended Drug Particles in the Formulation (number of particles/mL) | R-squared Value |
|---|---|---|---|---|---|---|
| Example 1 | 1.545 | 1.926 | 1.68 1 | 1.769 | 1.61E+ 10 | *0.955* |
| Comparative Example 1 | 1.343 | 2.213 | 2.60 1 | 1.643 | 4.29E+ 10 | *0.385* |

(continued)

| | Dissolved Drug MMAD (microns) | Dissolved Drug GSD | Suspended Drug MMAD (microns) | Suspended Drug GSD | Concentration of Suspended Drug Particles in the Formulation (number of particles/mL) | R-squared Value |
|---|---|---|---|---|---|---|
| Examples 2 | 1.295 | 1.888 | 1.682 | 1.638 | 5.03E+09 | 0.841 |
| Comparative Examples 2 | 1.368 | 2.015 | 2.497 | 1.775 | 1.31E+09 | 0.487 |

Table 6: ACI Results for Examples 3 and 4

| Test Component | Example 3: Beclomethasone Dipropionate | Example 4: Beclomethasone Dipropionate |
|---|---|---|
| Valve Stem | 2.60 | 2.57 |
| Actuator | 11.56 | 10.68 |
| Throat (USP Inlet) | 15.75 | 14.68 |
| Jet for Stage 0 | 0.35 | 0.44 |
| Plate 0 | 0.83 | 1.04 |
| Plate 1 | 0.36 | 0.90 |
| Plate 2 | 0.58 | 1.22 |
| Plate 3 | 2.44 | 2.94 |
| Plate 4 | 4.76 | 3.61 |
| Plate 5 | 5.26 | 3.90 |
| Plate 6 | 1.81 | 2.76 |
| Plate 7 | 0.58 | 2.17 |
| Filter | 1.14 | 2.98 |

Table 7: ACI Results for Examples 5 and 6

| Test Component | Example 5: Flunisolide Hemihydrate | Example 6: Flunisolide Hemihydrate |
|---|---|---|
| Valve Stem | 4.57 | 6.70 |
| Actuator | 19.44 | 23.20 |
| Throat (USP Inlet) | 24.67 | 23.69 |
| Jet for Stage 0 | 0.35 | 0.33 |
| Plate 0 | 0.81 | 0.84 |
| Plate 1 | 0.42 | 1.27 |
| Plate 2 | 0.49 | 2.21 |
| Plate 3 | 2.74 | 6.58 |
| Plate 4 | 9.45 | 7.09 |
| Plate 5 | 18.32 | 9.93 |
| Plate 6 | 9.31 | 8.57 |
| Plate 7 | 3.90 | 4.72 |
| Filter | 4.32 | 5.26 |

Table 8: ACI Results for Comparative Examples 3 and 4

| Test Component | Comparative Example 3: Beclomethasone Dipropionate | *Comparative Example 6: Flunisolide Hemihydrate* (meg/dose) |
|---|---|---|
| Valve Stem | 2.35 | *5.33* |
| Actuator | 10.78 | *22.27* |
| Throat (USP Inlet) | 12.25 | *21.12* |
| Jet for Stage 0 | 0.17 | *0.27* |
| Plate 0 | 0.26 | *0.56* |
| Plate 1 | 0.12 | *0.27* |
| Plate 2 | 0.12 | *0.32* |
| Plate 3 | 0.27 | *1.72* |
| Plate 4 | 1.33 | *6.46* |
| Plate 5 | 7.55 | *20.30* |
| Plate 6 | 6.71 | *13.12* |
| Plate 7 | 3.30 | *5.42* |
| *Filter* | *3.48* | *4.97* |

Table 9

| | MMAD | GSD |
|---|---|---|
| Example 3 | 2.155 | 1.924 |
| Example 4 | 1.826 | 2.657 |
| Example 5 | 1.437 | 1.897 |
| Example 6 | 1.509 | 2.620 |
| Comparative Example 3 | 0.97 | 1.807 |
| Comparative Example 4 | 1.216 | 1.848 |

Simulation Model

In addition to the actual examples described above, a useful alternative to Anderson Cascade Impactor tests using a computerized model was developed to simulate the impact of formulation parameters on the size distribution of the drug emitted from an MDI. The model predicts the aerodynamic particle size distribution (APSD) emitted from MDIs using an algorithm that predicts the residual delivered aerosol based on knowledge of the formulation parameters and input size of the suspended particulate drug or excipient. This algorithm is not limited to the analysis of monodisperse suspended drug particles, but allows for polydisperse suspended or excipient particle suspended in the formulation, multiple suspended drugs to be included in the formulation, and allows for dissolved excipients or a dissolved drug to be included in the modeled formulation. The algorithm schematic set forth below was converted to Visual Basic code embedded in a Microsoft Excel spreadsheet in which the output of the algorithm is stored. The computer code can be generated without undue difficulty based on the algorithm.

Schematic 1.

The first step in utilizing the model is to input the formulation information. The necessary information is described in Schematic 1. The individual particle density (as opposed to the bulk powder density) is needed for suspended bulking agent or drug partcles. This can be estimated by several approaches including observing settling or creaming in blends of HFA-134a or HFA-227. Once the formulation information is provided, the second step is to determine the size distribution of the initial droplets. This can be done experimentally or empirical equations can be used to provide initial droplet sizes for the simulations. Once the formulation information and initial atomized size distribution are defined, individual droplets are modeled one at a time using Steps 3 through 7 in Schematic 1. The initial droplet size of the single droplet to be modeled is determined by selecting the droplet size in a statistically random fashion based on the input size distribution of initial droplets (Step 2). Once the size of the droplet is known, the number of suspended drug or excipient particles contained in the droplet is estimated (Step 4) using the Poisson statistical distribution and the procedure described by elsewhere. The size of any suspended drug or excipient particles in the formulation are then determined by selecting drug particle sizes from the input size distribution of the bulk drug or excipient powder (input in Step 1) in a statistically random fashion. The total volume of suspended drug or excipient particles is limited such that it cannot exceed the volume of the droplet (although this limit is almost never reached). Once the volume of the drug or excipient particles is known, the mass and volume of any dissolved drug and/or non-volatile excipient (e.g. surfactant) can be calculated based on the formulation details provided in Step 1. Thus by Step 6, the mass and volume of suspended drug or excipient particles, non-volatile excipient, and solubilized drug in the droplet are known.

From the information obtained in Step 6, the composition of the 'residual particle' that remains once the propellant and co solvent (if present) evaporate away is determined. This information is used to calculate the aerodynamic diameter of the residual particle. For residual particles with more than one suspended drug or excipient particle, the shape factor was calculated using data from previous literature using the simplifying assumptions that the particles were all the same size. For agglomerates with more than four particles, the particles were assumed to have a packing density factor of 0.741.

For purposes of this description, the MMAD is defined as the aerodynamic particle diameter at which 50% of the drug mass is associated with particles smaller than the size and 50% of particle mass is associated with particles larger than the size. This is sometimes defined as the Dso or the Dvso if the particle density is assumed to be 1 gram per milliliter. The GSD is defined below:

$$GSD = \sqrt{\frac{D_{84.1}}{D_{15.9}}}$$

While the terms MMAD and GSD are generally used for size distributions that are a single mode and follow a lognormal size distribution, in this description they are used to describe aerosols even though some of them were not lognormally distributed. They are used simply to provide general information about the emitted size distribution.

With regard to drug combination MDIs, the degree of co-deposition from an MDI with one drug in solution and one in suspension has been determined to be highest for formulations containing large numbers of suspended drug particles. This can be done by either adding more drug (which usually is not an option due to dose-response considerations) or by using smaller micronized drug particles. The computer simulations described below indicate that good co-deposition is obtained for MDI formulations with more than about $3\times10^9$ suspended particles per milliliter. Preferably, the formulations in this invention contain more than $1\times10^{10}$ suspended particles per milliliter. The number of 15 particles per milliliter can be estimated using the equations below.

$$C_P = \frac{6C_D\sqrt{\rho}\exp(4.5\ln^2 GSD)}{\pi(MMAD)^3}$$

Equation 1

$$C_P = \frac{6C_D\exp(4.5\ln^2 GSD)}{\rho\pi(MMD)^3}$$

Equation 2

where:

- $\rho_p$ is the particle density (in grams per milliliter)

- MMAD is the mass median aerodynamic diameter of the micronized drug to be suspended in the formulation (in centimeters to maintain consistent units)

- MMD is the mass median diameter of the micronized drug to be suspended in the formulation (in centimeters to maintain consistent units)

- GSD is the geometric standard deviation of the micronized drug to be suspended in the formulation (dimensionless)

- $C_D$ is the mass concentration of drug in the formulation (in grams per milliliter)

- $C_p$ is the concentration of particles in the formulation (in particles per milliliter)

### Simulation Model Example

The algorithm described above was used to theoretically simulate the residual size distributions emitted for ten different MDI formulations of a drug in solution and a particulate bulking agent. For each formulation, a total of 20,000 particles were simulated. The aerodynamic size and mass of drug for each residual particle was calculated using the algorithm. After the size and drug mass was calculated for each of the 20,000 droplets, then the overall size distribution of the formulation was determined. Subsequently from this size distribution, the MMAD and GSD of the distribution were determined. The formulations simulated are described in Table 10.

Table 10.

| Formulation # | Dissolved Drug Conc (%) | Solid Particle Bulking Agent (%) | MMAD of Bulking Agent microns | GSD of Bulking Agent | Propellant | Ethanol Conc (%) |
|---|---|---|---|---|---|---|
| 1 | 0.03 | 0 | N/A | N/A | HFA-134a | 8 |
| 2 | 0.03 | 0.1 | 3.0 | 1.6 | HFA-134a | 8 |
| 3 | 0.03 | 1.0 | 3.0 | 1.6 | HFA-134a | 8 |
| 4 | 0.03 | 0.1 | 0.5 | 1.6 | HFA-134a | 8 |
| 5 | 0.03 | 1.0 | 0.5 | 1.6 | HFA-134a | 8 |

(continued)

| Formulation # | Dissolved Drug Conc (%) | Solid Particle Bulking Agent (%) | MMAD of Bulking Agent microns | GSD of Bulking Agent | Propellant | Ethanol Conc (%) |
|---|---|---|---|---|---|---|
| 6 | 0.30 | 0 | N/A | 1.6 | HFA-134a | 8 |
| 7 | 0.30 | 0.1 | 3.0 | 1.6 | HFA-134a | 8 |
| 8 | 0.30 | 1.0 | 3.0 | 1.6 | HFA-134a | 8 |
| 9 | 0.30 | 0.1 | 0.5 | 1.6 | HFA-134a | 8 |
| 10 | 0.30 | 1.0 | 0.5 | 1.6 | HFA-134a | 8 |

The calculated MMAD and GSD for the 10 formulations simulated are shown in Table 11, below. Formulation #1 is a comparative example to Formulations #2 to 5 and Formulation #6 is a comparative example to Formulations #7 to 10. The solid particle bulking agents were able to modify both the MMAD and GSD of the residual particle size distribution of the solubilized drug emitted from the MDI. The solid bulking agents that had the smaller particle size distribution were more effective at increasing the MMAD of the emitted drug than the larger solid particle bulking agents. For example, the 0.5 micron bulking agents were able to increase the MMAD nearly as much or even more at a concentration of 0.1% than the 3.0 micron bulking agent was at a concentration of 1.0%. On the other hand, the larger bulking agent was able to provide much more dramatic increases in the GSD.

Table 11.

| Formulation Number | Solubilized Drug | Solid Bulking Agent Concentration | | | Resultant Drug Particle Size Distribution | |
|---|---|---|---|---|---|---|
| | Conc. | Conc. | MMAD | GSD | MMAD | GSD |
| 1 | 0.03 | 0 | N/A | N/A | 0.63 | 1.78 |
| 2 | 0.03 | 0.1 | 3 | 1.6 | 0.64 | 1.92 |
| 3 | 0.03 | 1.0 | 3 | 1.6 | 0.94 | 2.99 |
| 4 | 0.03 | 0.1 | 0.5 | 1.6 | 0.91 | 1.85 |
| 5 | 0.03 | 1.0 | 0.5 | 1.6 | 1.71 | 1.86 |
| 6 | 0.30 | 0 | N/A | N/A | 1.36 | 1.78 |
| 7 | 0.30 | 0.1 | 3 | 1.6 | 1.42 | 1.84 |
| 8 | 0.30 | 1.0 | 3 | 1.6 | 1.60 | 2.13 |
| 9 | 0.30 | 0.1 | 0.5 | 1.6 | 1.89 | 2.00 |
| 10 | 0.30 | 1.0 | 0.5 | 1.6 | 1.94 | 1.74 |

The formulations that used the smaller (0.5 micron MMAD) bulking particles had size distributions that were quite lognormally distributed, whereas the formulations that used the larger (3.0 micron MMAD) bulking particles had size distributions that were much broader and were in some cases bimodal in nature.

**Claims**

1. A metered dose inhaler containing an aerosol formulation comprising:

   propellant;
   one or more drugs, wherein all the drug in the formulation is in dissolved form; and
   at least one non-drug bulking agent in undissolved particulate form suspended in the formulation.

2. The metered dose inhaler of claim 1, wherein the drug is selected from the group consisting of beclomethasone dipropionate, fluticasone propionate, budesonide, mometasone, ciclesonide, flunisolide, ipratropium, and tiotropium, including any physiologically acceptable salt, solvate, or enantiomer of any of the foregoing.

3. The metered dose inhaler of claims 1 or 2, where the drug completely dissolved in the formulation is present at a concentration of at least 0.01% by weight of the formulation.

4. The metered dose inhaler of claims 1 to 3, wherein the propellant includes a compound selected from the group consisting of 1,1,1,2-tetrafluoroethane (HFC-134a), 1,1,1,2,3,3,3-heptafluoropropane (HFC-227), and mixtures thereof.

5. The metered dose inhaler of claims 1 to 4, wherein the bulking agent is selected from the group consisting of lactose, DL-alanine, ascorbic acid, glucose, sucrose, D(+)trehalose.

6. The metered dose inhaler of claim 1 to 5, further comprising ethanol.

7. The metered dose inhaler of claim 1 to 6, further comprising a dispersing aid.

8. The metered dose inhaler of claim 1 to 7, wherein the non-drug bulking agent has a mass median diameter (MMD) of at least 2 microns.

9. The metered dose inhaler of claims 1-8, further comprising a dissolved low-volatility excipient.

10. The metered dose inhaler of claims 1 to 9, wherein the drug is tiotropium.

11. The metered dose inhaler of claims 2 to 9, wherein the drug is ipratropium.

12. The metered dose inhaler of claims 5 to 11, wherein the bulking agent is lactose.


**Patentansprüche**

1. Ein Dosierinhalator, enthaltend eine Aerosolformulierung, umfassend:

   Treibgas;
   einen oder mehrere Arzneistoffe, wobei sämtliche Arzneistoffe in der Formulierung in gelöster Form vorliegen; und
   mindestens einen nicht-medikamentösen Füllstoff in Form ungelöster Partikel, suspendiert in der Formulierung.

2. Der Dosierinhalator nach Anspruch 1, wobei der Arzneistoff ausgewählt ist aus der Gruppe, bestehend aus Beclomethasondipropionat, Fluticasonpropionat, Budesonid, Mometason, Ciclesonid, Flunisolid, Ipratropium und Tiotropium, einschließlich jedes physiologisch verträglichen Salzes, Solvats oder Enantiomers jedes der vorgenannten Stoffe.

3. Der Dosierinhalator nach Anspruch 1 oder 2, wobei der Arzneistoff, der vollständig in der Formulierung gelöst ist, in einer Konzentration von mindestens 0,01 Gew.-% vorhanden ist.

4. Der Dosierinhalator nach den Ansprüchen 1 bis 3, wobei das Treibgas eine Verbindung enthält, die aus der Gruppe, bestehend aus 1,1,1,2-Tetrafluorethan (HFC-134a), 1,1,1,2,3,3,3-Heptafluorpropan (HFC-227) und Mischungen davon, ausgewählt ist.

5. Der Dosierinhalator nach den Ansprüchen 1 bis 4, wobei der Füllstoff aus der Gruppe, bestehend aus Laktose, DL-Alanin, Ascorbinsäure, Glucose, Sucrose, D(+)-Trehalose, ausgewählt ist.

6. Der Dosierinhalator nach den Ansprüchen 1 bis 5, ferner umfassend Ethanol.

7. Der Dosierinhalator nach den Ansprüchen 1 bis 6, ferner umfassend ein Dispergierhilfsmittel.

8. Der Dosierinhalator nach den Ansprüchen 1 bis 7, wobei der nicht-medikamentöse Füllstoff einen massenmedianen Durchmesser (MMD) von mindestens 2 Mikrometer aufweist.

**9.** Der Dosierinhalator nach den Ansprüchen 1 bis 8, ferner umfassend einen gelösten Exzipienten mit geringer Flüchtigkeit.

**10.** Der Dosierinhalator nach den Ansprüchen 1 bis 9, wobei der Arzneistoff Tiotropium ist.

**11.** Der Dosierinhalator nach den Ansprüchen 2 bis 9, wobei der Arzneistoff Ipratropium ist.

**12.** Der Dosierinhalator nach den Ansprüchen 5 bis 11, wobei der Füllstoff Laktose ist.

**Revendications**

**1.** Inhalateur doseur contenant une formulation d'aérosol comprenant :

un propulseur ;
un ou plusieurs médicaments, la totalité du médicament dans la formulation étant sous forme dissoute ; et
au moins un agent de charge non-médicament sous forme particulaire non dissoute, en suspension dans la formulation.

**2.** Inhalateur doseur selon la revendication 1, dans lequel le médicament est choisi dans le groupe constitué par le dipropionate de béclométhasone, le propionate de fluticasone, le budésonide, la mométasone, le ciclésonide, la flunisolide, l'ipratropium, et le tiotropium, y compris n'importe quel sel, solvate ou énantiomère physiologiquement acceptable de l'un quelconque des précédents.

**3.** Inhalateur doseur selon la revendication 1 ou 2, dans lequel le médicament complètement dissous dans la formulation est présent à une concentration d'au moins 0,01 % en poids de la formulation.

**4.** Inhalateur doseur selon les revendications 1 à 3, dans lequel le propulseur comprend un composé choisi dans le groupe constitué par le 1,1,1,2-tétrafluoroéthane (HFC-134a), le 1,1,1,2,3,3,3-heptafluoropropane (HFC-227), et leurs mélanges.

**5.** Inhalateur doseur selon les revendications 1 à 4, dans lequel l'agent de charge est choisi dans le groupe constitué par le lactose, la DL-alanine, l'acide ascorbique, le glucose, le saccharose, le D(+)-tréhalose.

**6.** Inhalateur doseur selon les revendications 1 à 5, comprenant en outre de l'éthanol.

**7.** Inhalateur doseur selon les revendications 1 à 6, comprenant en outre un auxiliaire de dispersion.

**8.** Inhalateur doseur selon les revendications 1 à 7, dans lequel l'agent de charge non-médicament a un diamètre médian en masse (MMD) d'au moins 2 micromètres.

**9.** Inhalateur doseur selon les revendications 1 à 8, comprenant en outre un excipient faiblement volatil dissous.

**10.** Inhalateur doseur selon les revendications 1 à 9, dans lequel le médicament est le tiotropium.

**11.** Inhalateur doseur selon les revendications 2 à 9, dans lequel le médicament est l'ipratropium.

**12.** Inhalateur doseur selon les revendications 5 à 11, dans lequel l'agent de charge est le lactose.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9965464 A
- US 7018618 B
- US 7601336 B
- US 6716414 B
- US 6451285 B
- US 6315985 B
- US 6290930 B
- US 6045778 A
- US 6004537 A
- US 5776433 A
- US 5676930 A
- US 20030147814 A
- US 20030152521 A
- US 20050267145 A
- WO 02066422 A
- WO 02070490 A
- WO 02076933 A
- WO 03024439 A
- WO 03072539 A
- WO 03091204 A
- WO 04016578 A
- WO 2004022547 A
- WO 2004037807 A
- WO 2004037773 A
- WO 2004037768 A
- WO 2004039762 A
- WO 2004039766 A
- WO O1142193 A
- WO 03042160 A
- WO 03082827 A
- WO O1110143 A
- WO 9854159 A
- WO 04005229 A
- WO 04009016 A
- WO 04009017 A
- WO 04018429 A
- WO 031104195 A
- WO 03082787 A
- WO 03082280 A
- WO 03059899 A
- WO 031101932 A
- WO 0202565 A
- WO 01116128 A
- WO 0066590 A
- WO 03086294 A
- WO 04026248 A
- WO 03061651 A
- WO 0308277 A
- WO 9313055 A
- WO 9830537 A
- WO 0250021 A
- WO 9534534 A
- WO 9962875 A
- WO 0226722 A
- US 5552438 A
- WO 9916766 A
- WO 9947505 A
- WO 04024728 A
- EP 2003014867 W
- EP 2004005494 W
- WO O104118 A
- US 2004081627 A

**Non-patent literature cited in the description**

- **JOHNSON, M.** Inhaled corticosteroid -long-acting b2-agonist synergism: therapeutic implications in human lung disease. *Proceedings of Respiratory Drug Delivery,* 2004, vol. IX, 99-108
- **HOFGEN, N. et al.** 15th EFMC lnt Symp Med Chern. 06 September 1998
- *CHEMICAL ABSTRACTS,* 247584020-9
- **LANDELLS, L.J. et al.** Annu Cong Eur Resp Soc. *Eur Resp J,* 19 September 1998, vol. 12 (28
- *CHEMICAL ABSTRACTS,* 162401-32-3
- **TANABE SEIYAKU ; FUJI, K. et al.** *J Pharmacol Exp Ther,* 1998, vol. 284 (1), 162
- *CHEMICAL ABSTRACTS,* 22254-24-6
- *CHEMICAL ABSTRACTS,* 30286-75-0
- *CHEMICAL ABSTRACTS,* 136310-93-5
- *CHEMICAL ABSTRACTS,* 262586-79-8
- *CHEMICAL ABSTRACTS,* 28797-61-7
- *CHEMICAL ABSTRACTS,* 133099-04-4
- *CHEMICAL ABSTRACTS,* 133099-07-7
- *CHEMICAL ABSTRACTS,* 5633-20-5
- *CHEMICAL ABSTRACTS,* 15793-40-5
- *CHEMICAL ABSTRACTS,* 124937-51-5
- *CHEMICAL ABSTRACTS,* 12493752-6
- *CHEMICAL ABSTRACTS,* 26095-59-0
- *CHEMICAL ABSTRACTS,* 10405-02-4
- *CHEMICAL ABSTRACTS,* 242478-37-1
- *CHEMICAL ABSTRACTS,* 242478-38-2

• **S.W. AAPS.** *PharmSciTech,* 2008, vol. 19, 112-115